(19) Europäisches Patentamt
European Patent Office
Office européen des brevets

(11) Veröffentlichungsnummer: **0 151 967**
**B1**

(12) **EUROPÄISCHE PATENTSCHRIFT**

(45) Veröffentlichungstag der Patentschrift: **23.08.89**

(51) Int. Cl.⁴: **C 07 C 103/56,** C 07 C 103/64, C 07 C 102/00

(21) Anmeldenummer: **85100604.9**

(22) Anmeldetag: **22.01.85**

(54) **Verfahren zur Herstellung von N-substituierten, alpha, beta-ungesättigten Carbonsäureamiden.**

(30) Priorität: **26.01.84 DE 3402599**

(43) Veröffentlichungstag der Anmeldung: **21.08.85 Patentblatt 85/34**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen:
**EP-A-0 013 416**
**EP-A-0 070 425**
**DE-A-2 623 838**
**DE-A-2 836 520**

(73) Patentinhaber: **Chemische Fabrik Stockhausen GmbH, Bäkerpfad 25, D-4150 Krefeld (DE)**

(72) Erfinder: **Dahmen, Kurt, Dr. Dipl.- Chem., Von-Velsen- Strasse 6, D-4050 Mönchengladbach 2 (DE)**
Erfinder: **Küster, Erich, Dr. Dipl.- Chem., Friedrich-Ebert- Strasse 4, D-4150 Krefeld (DE)**
Erfinder: **Mertens, Richard, Dr. Dipl.- Chem., Dahlerdyk 116 a, D-4150 Krefeld (DE)**
Erfinder: **Brehm, Helmut, Dipl.- Ing., Dachstrasse 22, D-4150 Krefeld (DE)**

(74) Vertreter: **Klöpsch, Gerald, Dr.- Ing., An Gross St. Martin 6, D-5000 Köln 1 (DE)**

**EP 0 151 967 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zur Herstellung von N-substituierten α,β-ungesättigten Carbonsäureamiden, bei dem als Ausganssubstanz das jeweilige α,β-ungesättigte, am Amidostickstoff unsubstituierte Carbonsäureamid eingesetzt wird.

N-substituierte α,β-ungesättigte Carbonsäureamide spielen auf vielen Gebieten der Technik eine erhebliche Rolle. So finden sich Vertreter dieser Stoffklasse beispielsweise unter den in der Kunststoffindustrie bedeutsamen Monomeren. N-substituierte α,β-ungesättigte Carbonsäureamide sind aber auch unter den Herbiziden zu finden.

Syntheseverfahren, die einen technisch und wirtschaftlich befriedigenden Zugang zu N-substituierten α,β-ungesättigten Carbonsäureamiden gestatten, sind daher seit langem Gegenstand chemischer Forschung und Entwicklung. Bisher ist es indes nicht gelungen, einen völlig nachteilsfreien Zugang zu N-substituierten α, β-ungesättigten Carbonsäureamiden zu erschließen.

Eine ausführliche Würdigung der Vor- und Nachteile bislang bekannter Syntheseverfahren findet sich in der europäischen Patentschrift 0 013 416.

Ausgehend etwa von α,β-ungesättigten Carbonsäureestern oder -nitrilen wird in den besten bislang bekannten Verfahren durch Anlagerung von Wasser, Alkoholen oder Aminen die reaktive Doppelbindung geschützt (die s.g. Michael-Addition - z. B. vgl. O. Bayer, Angew. Chem. 61 (1949) S. 229; Org. React. 10 (1959) S. 179). Diese Schutzgruppe wird dann nach einem oder mehreren Reaktionsschritten unter Rückbildung der Doppelbindung abgespalten. Die Abspaltungsreaktion kann thermisch, ggfs. unter Zusatz von Katalysatoren durchgeführt werden.

Eine Reaktionsfolge dieser Art, bei der allerdings vom großtechnisch leicht zugänglichen Acrylamid ausgegangen wird, beschreibt das europäische Patent Nr. 0 070 425. In einer Einstufenreaktion wird an die Kohlenstoff-Doppelbindung ein primäres oder sekundäres Amin angelagert, anschliessend mit einem Überschuß an Amin (analog zum entsprechenden Verfahren mit β-Hydroxy- bzw. β-Alkoxypropionamiden, wenn die Doppelbindung durch Wasser oder ein Alkanol geschützt wird) unter Abspaltung von Ammoniak umamidiert und dann unter weiterer Erhöhung der Temperatur im Vakuum die Amino-Schutzgruppe wieder abgespalten, wobei das gewünschte N-substituierte Acrylamid entsteht. Als Nachteil dieser mehrstufigen Reaktionsführung ist anzusehen, daß, wie die Beispiele zeigen, die Aminkomponente in hohem (etwa in dreifachem) Überschuß angewendet werden muß. Bei diesen Aminkomponenten handelt es sich aber, gerade wenn die technisch besonders interessanten N-substituierten α,β-ungesättigte Carbonsäureamide hergestellt werden sollen, um relativ teure Verbindungen. Dies ist zum Beispiel der Fall beim 3-Dimethylaminopropylamin und beim N',N',-3,3-Tetra-methylpropandiamin-1,3. In derartigen Fällen ist der Einsatz des Verfahrens mit nicht zu unterschätzenden wirtschaftlichen Nachteilen verbunden, zumal eine unmittelbare Wiederverwendung des Amins angesichts der Nebenreaktionen, die bei den erhöhten Temperaturen (200 bis 300° C) des letzten Reaktionsschritts auftreten können (Hofmann und Cope-Eliminierung), mit Schwierigkeiten verbunden ist.

In den DE-OS-2 623 838 und 2 836 520 wird bereits die Herstellung von N-substituierten β-Alkoxipropionamiden und deren Zersetzung zu N-substituierten (Meth)-Acrylamiden beschrieben.

Werden kurzkettige Alkohole als Schutzgruppe verwendet, so lassen sich im Gegensatz zur Lehre der DE-OS-2 623 838 die N-substituierten β-Alkoxipropionamide nur unzersetzt destillieren. Erst bei höheren Temperaturen tritt eine merkliche Spaltung ein. Bedingt durch die hohen Pyrolysetemperaturen wird die Bildung von Nebenprodukten gefördert und die Ausbeute an Produkt entsprechend gesenkt.

Die DE-OS-2 836 520 beschreibt die Verwendung von basischen Katalysatoren im Pyrolyseschritt, wodurch die Pyrolysetemperatur auf ca. 100°C gesenkt werden kann. Trotzdem zeigt auch dieses Verfahren noch Nachteile. Die basischen Katalysatoren initiieren anionische Nebenreaktionen - speziell bei den Acrylamidderivaten treten anionische Kondensationen bzw. Polymerisationen auf - und bewirken dadurch unerwünschte Ausbeuteverluste.

Versuche, langkettige Alkohole, wie z. B. Hexanol, als Schutzgruppe zu verwenden, führen im Fall der Michael-Addition an die α,β-ungesättigten Carbonsäureamide nur zu geringen Umsätzen. Bei der entsprechenden Anlagerung an die α,β-ungesättigten Carbonsäureester entsteht durch zusätzliche Umesterung ein Gemisch von mindestens vier Produkten, das die weitere Reaktionsführung mit erheblichen Trennproblemen belastet.

Aufgabe der vorliegenden Erfindung ist daher, ein Verfahren, das es gestattet, ohne störende Nebenreaktionen unter Ausnutzung der Schutzwirkung von Alkoholen für Kohlenstoff-Doppelbindungen durch Umamidierung und nachfolgende Pyrolyse zu N-substituierten α,β-ungesättigten Carbonsäureamiden zu gelangen. Hierfür ist es erforderlich, daß die alkoholische Schutzgruppe leicht und vollständig mit dem unsubstituierten, α,β-ungesättigten Carbonsäureamid umgesetzt und nach der Umamidierung ohne Katalysatoren und bei möglichst niedrigen Temperaturen unter Bildung der gewünschten N-substituierten Carbonsäureamide wieder abgespalten werden kann.

Diese Aufgabe wird erfindungsgemäß dadurch gelöst, daß man das am Amidstickstoff unsubstituierte, α,β-ungesättigte Carbonsäureamid unter Basenkatalyse nach der Michael-Reaktion mit einer organischen, wenigstens zwei alkoholische OH-Gruppen enthaltenden Polyhydroxiverbindung mit einem Siedepunkt von ≧ 150°C bei 1010 hPa umsetzt, das entstandene Michael-Addukt mit einem primären oder sekundären Amin unter Ammoniakeliminierung zum N-substituierten Carbonsäureamid mit geschützter Doppelbindung umsetzt und aus diesem Produkt anschliessend bei erhöhter Temperatur die Polyhydroxyverbindung unter Bildung des N-

substituierten α,β-ungesättigten Carbonsäureamids eliminiert.

Überraschenderweise hat sich gezeigt, daß mehrwertige Alkohole mit einem Siedepunkt von ≧ 150°C bei 1010 hPa (= 760 Torr) sich leicht an α,β-ungesättigte Carbonsäureamide wie (Meth)-Acrylamid anlagern und auch leicht und vollständig abspalten lassen. Die basenkatalysierte Addition dieser Polyhydroxyverbindungen an das am Amidostickstoff unsubstituierte α,β-ungesättigte Carbonsäureamid läuft ohne nennenswerte Nebenreaktionen schnell und vollständig ab, ebenso die Rückspaltung.

Als Polyhydroxyverbindungen, die wenigstens 2 alkoholische OH-Gruppen enthalten müssen, sind die aliphatischen mehrwertigen Alkohole, die grad- oder verzweigtkettig sein können besonders geeignet. Bevorzugt werden aliphatische Diole mit 2 bis 6 Kohlenstoffatomen, z. B. 1,2-Ethan-diol. Ebenso geeignet sind die aliphatischen Triole mit 3 bis 6 C-Atomen, z. B. Glyzerin oder Trimethylolpropan. Auch solche mehrwertigen Alkohole, die Heteroatome enthalten, wie Aminoalkohole oder auch Etherbrücken enthaltende wenigstens zweiwertige Alkohole sind geeignet. Beispiele für Aminoalkohole sind Triethanolamin, Beispiele für Etheralkohole, die Di-hydroxyether, insbesondere Di-(2)hydroxyethyl)ether.

Die in der Regel leicht exotherme Addition der Polyhydroxyverbindung an das Carbonsäureamid wird zweckmäßigerweise bei einer Temperatur bis höchstens zum Siedepunkt der Polyhydroxyverbindung, vorzugsweise im Bereich von 20 bis 70, besonders bevorzugt von 40 bis 50°C durchgeführt, wobei das Molverhältnis von Polyhydroxiverbindung und α,β-ungesättigten Ausgangscarbonsäureamid 0,75 : 1 bis 2 : 1 beträgt. Bevorzugt werden äquimolare Mengen eingesetzt.

Die Michaeladdition läuft unter basischer Katalyse mit Alkalihydroxiden oder Alkalialkoholaten ab. Auch quartäre Ammoniumhydroxide von der Art des Benzyltrimethylammoniumhydroxids (Triton B) sowie basische Ionenaustauscher sind als Katalysatoren geeignet. Die Umsetzung erfolgt praktisch quantitativ. Ein Zusatz von Polymerisationsinhibitoren ist möglich, in der Regel aber nicht unbedingt notwendig.

Als Ausgangscarbonsäureamide kommen prinzipiell alle α,β-ungesättigten Verbindungen in Frage. Besonders bevorzugt werden Carbonsäureamide der allgemeinen Formel

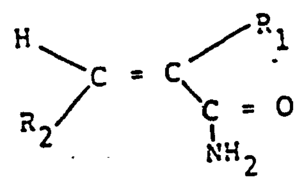

in der $R_1$ und $R_2$ Wasserstoff oder Methyl bedeuten, typische Beispiele sind Acrylamid, Methacrylamid und Crotonamid.

Das Michael-Addukt wird zweckmäßigerweise nicht isoliert, sondern sofort mit einem primären oder sekundären bevorzugt aliphatischen Amin umamidiert.

Für das erfindungsgemäße Verfahren sind prinzipiell auch alle primären und sekundären Amine geeignet, bevorzugt werden Amine der allgemeinen Formel

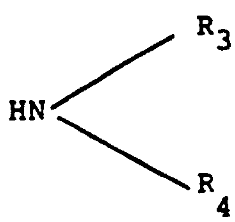

verwendet, in der

entweder

$R_3$ Wasserstoff und

$R_4$ den Rest -Q-$R_5$ bedeuten, wobei

Q für einen gegebenenfalls substituierten gerad- oder verzweigtkettigen oder aus 5-6-gliedrigen iso- oder heterocyclischen, vorzugsweise mit Stickstoff- und/oder Sauerstoffatomen als Ringglieder, Ringen bestehenden oder solche Ringe enthaltenden organischen Rest mit 2 bis 18 C-Atomen steht

oder

$R_3$ und $R_4$ jeweils einen Alkylrest mit 1 bis 5 Kohlenstoffatomen bedeuten, wobei $R_3$ und $R_4$ auch zusammen einen 5- oder 6-gliedrigen iso- oder heterocyclischen Ring bilden können und

wobei $R_5$ für Wasserstoff, eine Dialkylaminogruppe oder Alkoxigruppe steht, deren Alkylreste jeweils 1 - 4 Kohlenstoffatome enthalten.

Typische Vertreter von Aminen, die unter die allgemeine Formel $HNR_3R_4$ fallen, gehorchen den allgemeinen Formeln

$H-NH-(Q)-R^6$ , 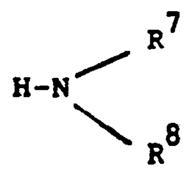

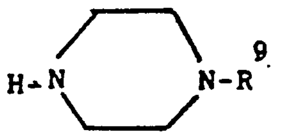 oder 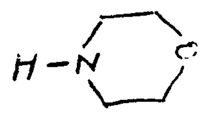

worin

Q die oben erwähnte Bedeutung hat,

$R_6$ wie $R_5$, $R_7$, $R_8$ die gleiche Bedeutung wie $R_3$ und $R_4$ haben und

$R_9$ einen Alkylrest mit 1 bis 4 Kohlenstoffatomen bedeutet.

Geeignet sind primäre Amine, etwa Methylamin, Ethylamin n-Butylamin, 2-Ethylhexylamin, Cyclohexylamin, Methoxipropylamin, vorzugsweise N,N-Dimethyl- und N,N-2,2-Tetramethylpropandiamin-1,3 sowie N,N-Dimethyl-ethandiamin-1,2; von den sekundären Aminen seien stellvertretend Dimethylamin, Dibutylamin, Morpholin und N-Methylpiperazin genannt. Die Umamidierung, die, wie bereits erwähnt, vorteilhafterweise im gleichen Gefäß wie die Michael-Addition erfolgt, wird vorteilhafterweise in Gegenwart katalytischer Mengen - im Regelfall 1 bis 5 Mol % - einer Carbonsäure, wie Ameisen-, Essig-, Propion- bzw. Buttersäure durchgeführt. Diese Säure dient auch der Neutralisierung des basischen Katalysators der Michael-Addition. Vorzugsweise wird Acrylsäure verwendet. Anorganische Säuren eignen sich im Prinzip als Katalysatoren, führen jedoch bei der nachfolgenden Pyrolyse in der Regel zu Schwierigkeiten.

Bei der Umamidierung wird das primäre oder sekundäre Amin in der Regel im Molverhältnis 1 : 1 bis 1,5 : 1 bezogen auf das am Amidstickstoff unsubstituierte α,β-ungesättigte Carbonsäureamid eingesetzt. Die Umsätze liegen bei dieser Reaktion in der Regel höher als 90 % der Theorie. Ein eventueller Überschuß des primären oder sekundären Amins kann vor der Pyrolyse abdestilliert werden. Um die Nebenreaktionen zu vermeiden, die durch die Bildung von Aminooxiden hervorgerufen werden können, empfiehlt es sich, sowohl bei der Umamidierung als auch bei der nachfolgenden Pyrolyse unter Inertgas, z. B. Stickstoff, zu arbeiten. Aminooxide neigen dazu, bei erhöhter Temperatur den Cope-Abbau zu erleiden, durch den eine weitere Doppelbindung im Molekül entsteht, die bei einer nachfolgenden Polymerisation als Vernetzer wirkt, so daß nicht wasserlösliche Polymere entstehen würden.

Die Umamidierungsreaktion wird zweckmäßig bei Temperaturen im Bereich von 100 bis 180°C, vorzugsweise 130 bis 170°C, durchgeführt.

Die so erhaltenen N-substituierten β-gesättigten Propionamide werden in einem Vakuum von 10 bis 100 hPa und Temperaturen von mindestens 150, vorzugsweise 150 bis 300°C, pyrolitisch zersetzt, wobei sich die bevorzugte Pyrolysetemperatur nach der Art des darzustellenden N-substituierten α,β-ungesättigten Carbonsäureamids richtet. Vorzugsweise liegt die Pyrolysetemperatur indes bei 170 bis 210°C. In diesem Temperaturbereich sind etwa N-(N',N'-Dimethyl-3-ami-nopropyl)acrylamid bzw. N-(N',N',2,2-Tetramethyl-3-aminopropyl)acrylamid in hohen Ausbeuten pyrolytisch zugänglich.

Auch bei dieser Reaktion kann eine Polymerisationsinhibitorzugabe erfolgen. Sie ist jedoch vielfach nicht notwendig.

Falls ein Polymerisationsinhibitor zugegeben werden muss, empfiehlt sich die Verwendung von nicht flüchtigen Substanzen wie Kupferpulver oder organischen oder anorganischen Kupfer(II)salzen. Es ist empfehlenswert, die Fraktionierung des Produktgemischs über eine Trennkolonne ebenfalls in Vakuum durchzuführen. Die Dimensionierung der Trennkolonne richtet sich nach der Art des mehrwertigen Alkohols und des herzustellenden N-substituierten α,β-ungesättigten Carbonsäureamids.

Pyrolyse und Fraktionierung sind sowohl in Kombination als auch in zwei aufeinanderfolgenden Verfahrensschritten vorzunehmen. Eine Gefahr der Rückreaktion der mehrwertigen Alkohole mit der gebildeten Doppelbindung besteht nicht, da diese Reaktion ohne starke basische Katalyse nicht abläuft. Wenn Amine, etwa gemäß EP-0 070 425 als Schutzgruppen verwendet werden, tritt die Rückreaktion nachteilig in Erscheinung und bewirkt Ausbeuteverringerungen, die beim Verfahren gemäß der Erfindung wirksam vermieden werden.

Das Verfahren gemäß der Erfindung stellt einen neuen verlustarmen Zugang unter anderem zu N-substituierten (Meth)-Acrylamiden dar, die als wertvolle Monomeren bei der Herstellung kationischer Homo- bzw. Copolymeren Verwendung finden. Diese Homo- bzw. Copolymeren können mannigfaltig eingesetzt werden.

Die folgenden Beispiele erläutern die Erfindung ohne eine Einschränkung der Anwendbarkeit des Verfahrens anzudeuten:

**Beispiel 1:**

N-(N',N'-Dimethyl-3-aminopropyl)acrylamid (DIMAPA)

Man erwärmt 186,2 g (3,0 Mol) 1,2-Ethandiol auf 45 bis 55°C, fügt 2 g (0,03 Mol) 85-%-iges Kaliumhydroxid zu und versetzt portionsweise mit 213,2 g (3,0 Mol) kristallinem Acrylamid. Nach dessen Auflösung rührt man 3 Stunden bei 50°C nach. Man neutralisiert mit 6,5 g (0,09 Mol) Acrylsäure und gibt unter Rühren 460 g (4,5 Mol) N,N-Dimethylpropandiamin-1,3 zu und erhitzt unter Stickstoffdurchleiten 6 bis 8 Stunden über ein Temperaturintervall von 130 bis 170°C bis zum Ende der Ammoniakentwicklung. Das Reaktionsprodukt wird zur Pyrolyse in dem Maße in einen auf 200 bis 210°C geheizten Kolben eingespeist, wie über eine Kolonne bei 30 hPa Druck seitlich DIMAPA und über Kopf das abgespaltene 1,2-Ethandiol und das überschüssige Amin abgezogen werden. Die DIMAPA-Fraktion ergibt 428 g 93-%-iges Produkt mit Kp 30 = 140 bis 150°C, was einer Ausbeute von 85 % d.Th. entspricht.

NMR ($CD\,Cl_3$) : δ = 1,55 bis 1,95 (m,2); 2,25 (s,6); 2,4 (t,2); 3,1 bis 3,55 (m,2); 5,45 bis 6,3 (m,3); 8,1 (m,1).

**Beispiel 2:**

N-(N',N',2,2-Tetramethyl-3-aminopropyl)-acrylamid (TEMAPA)

Man erwärmt 186,2 g (3,0 Mol) 1,2-Ethandiol auf 45 bis 55°C, fügt 2 g (0,03 Mol) 85-%-iges Kaliumhydroxid zu und versetzt portionsweise mit 213,2 g (3,0 Mol) kristallinem Acrylamid. Nach dessen Auflösung rührt man bei 50°C nach. Man neutralisiert mit 6 g (0,1 Mol) Eisessig und gibt unter Rühren 586 g (4,5 Mol) N,N,2,2-Tetramethyl-propandiamin-1,3 zu und erhitzt 6 bis 8 Stunden über ein Temperaturintervall von 130 bis 170°C bis zum Ende der Ammoniakentwicklung. Das Reaktionsprodukt wird zur Pyrolyse in dem Maße in einen auf 190 bis 210°C geheizten Kolben eingespeist, wie über eine Kolonne bei 30 hPa Druck seitlich TEMAPA und über Kopf das abgespaltene 1,2-Ethandiol und das überschüssige Amin abgezogen werden. Die TEMAPA-Fraktion ergibt 523,7 g 95-%-iges Produkt mit Kp 30 = 135 bis 145°C, was einer Ausbeute von 90 % d.Th. entspricht.

NMR ($CD\,Cl_3$) : δ = 0,9 (s,6); 2,3 (m,8); 3,15 (d,2); 5,3 bis 6,5 (m,3); 8,0 (m,1).

**Beispiel 3:**

N-(N',N'-Dimethyl-2-aminoethyl)-acrylamid (DIMETA)

Man erwärmt 186,2 g (3,0 Mol) 1,2-Ethandiol auf 45 bis 55°C, fügt 6,2 g (0,015 Mol) 40-%-ige wäßrige Triton B-Lösung zu und versetzt portionsweise mit 213,2 g (3,0 Mol) kristallinem Acrylamid. Nach dessen Auflösung rührt man 3 Stunden bei 50°C nach. Man neutralisiert mit 6,5 g (0,09 Mol) Acrylsäure und gibt unter Rühren 397 g (4,5 Mol) N,N-Dimethylethandiamin-1,2 zu und erhitzt unter Stickstoffdurchleiten 6 bis 8 Stunden über ein Temperaturintervall von 125 bis 170°C bis zum Ende der Ammoniakentwicklung. Das Reaktionsprodukt wird zur Pyrolyse in dem Maße in einen 200 bis 210°C geheizten Kolben eingespeist, wie über eine Kolonne bei 30 hPa Druck seitlich DIMETA und über Kopf das abgespaltene 1,2-Ethandiol und das überschüssige Amin abgezogen werden. Die DIMETA-Fraktion ergibt 409 g 95-%-iges Produkt mit Kp 30 = 138 bis 146°C, was einer Ausbeute von 91 % d.Th. entspricht.

NMR ($CD\,Cl_3$) : δ = 2,23 (s,6); 2,45 (t,2); 3,3 (m,2); 5,4 bis 6,25 (m,3); 7,6 (m,1).

**Beispiel 4:**

N-(2-Ethylhexyl)-acrylamid

Man erwärmt 186,2 g (3,0 Mol) 1,2-Ethandiol auf 45 bis 55°C, fügt 2 g (0,03 Mol) 85-%-iges Kaliumhydroxid zu und versetzt portionsweise mit 213,2 g (3,0 Mol) kristallinem Acrylamid. Nach dessen Auflösung rührt man 3 Stunden bei 50°C nach. Man neutralisiert mit 6,5 g (0,09 Mol) Acrylsäure, gibt unter Rühren 582 g (4,5 Mol) 2-Ethylhexylamin zu und erhitzt unter Stickstoffdurchleiten 6 bis 8 Stunden über ein Temperaturintervall von 130 bis 170°C bis zum Ende der Ammoniakentwicklung. Das Reaktionsprodukt wird zur Pyrolyse in dem Maße in einen auf 190 bis 220°C geheizten Kolben gespeist, wie über eine Kolonne bei 30 hPa Druck seitlich N-(2-Ethylhexyl)-acrylamid und über Kopf das abgespaltene 1,2-Ethandiol und das überschüssige Amin abgezogen werden. Es werden 542 g N-(2-Ethylhexyl)-acrylamid (Kp 30 = 155 bis 160°C) in 95-%-iger Reinheit erhalten werden.

**Beispiel 5:**

N-Propylacrylamid

Man erwärmt 186,2 g (3,0 Mol) 1,2-Ethandiol auf 45 bis 55° C, fügt 2 g (0,03 Mol) 85-%-iges Kaliumhydroxid zu und versetzt portionsweise mit 213,2 g (3,0 Mol) kristallinem Acrylamid. Nach dessen Auflösung rührt man 3 Stunden bei 50° C nach. Man neutralisiert mit 6,5 g (0,09 Mol) Acrylsäure, gibt unter Rühren 266 g (4,5 Mol) n-Propylamin zu und erhitzt in einem Autoklaven bei 1 Bar Überdruck 6 bis 8 Stunden über ein Temperaturintervall von 130 bis 170° C. Das Reaktionsprodukt wird zur Pyrolyse in dem Maße in einen auf 180 bis 200° C geheizten Kolben gespeist, wie über eine Kolonne bei 66,5 hPa Druck seitlich N-Propylacrylamid und über Kopf das abgespaltene 1,2-Ethandiol und das überschüssige Amin abgezogen werden. Es wurden 351 g N-Propylacrylamid (Kp 66,5 = 138 bis 142° C) erhalten mit 4 % 1,2-Ethandiol als Nebenbestandteil.

**Beispiel 6:**

N-(N',N'-Dimethyl-3-aminopropyl)acrylamid (DIMAPA)

Man erwärmt 276,3 g (3,0 Mol) Glycerin auf 55 bis 65° C, fügt 4 g (0,06 Mol) 85-%-iges Kaliumhydroxid zu und versetzt portionsweise mit 213,2 g (3,0 Mol) kristallinem Acrylamid. Nach dessen Auflösung rührt man 5 Stunden bei 60° C, wonach der Monomerengehalt auf 0,8 % gesunken ist. Man gibt 14,5 g (0,2 Mol) Acrylsäure und 460 g (4,5 Mol) N,N-Dimethylpropandiamin-1,3 zu und erhitzt unter Stickstoffdurchleiten 8 bis 10 Stunden über ein Temperaturintervall von 130 bis 170° C bis zum Ende der Ammoniakentwicklung. Das Reaktionsprodukt wird zur Pyrolyse in dem Maße in einen auf 190 bis 210° C geheizten Kolben eingespeist, wie über eine Kolonne bei 30 hPa seitlich DIMAPA sowie Glycerin und über Kopf das überschüssige Amin abgezogen werden. Man erhält 645 g eines Produktes mit 58 % DIMAPA mit Kp 30 = 160 bis 170° C, was einer Ausbeute von 80 % d.Th. entspricht.

**Patentansprüche**

1. Verfahren zur Herstellung von N-substituierten α,β-ungesättigten Carbonsäureamiden durch Umsetzung eines am Amido-Stickstoff unsubstituierten α,β-ungesättigten Carbonsäureamids unter Basenkatalyse gemäß der Michael-Addition mit einem Alkohol, Umamidierung des entstandenen Michael-Addukts mit einem primären oder sekundären Amin unter Ammoniakeliminierung zum N-substituierten Carbonsäureamid mit geschützter Doppelbindung und mit nachfolgender pyrolytischer Alkoholabspaltung, dadurch gekennzeichnet, daß man die Michael-Addition mit einer organischen, wenigstens 2 alkoholische OH-Gruppen enthaltenden Polyhydroxyverbindung mit einem Siedepunkt von ≧ 150°C bei 1010 hPa im Molverhältnis 0,75 : 1 bis 2 : 1, bezogen auf das α,β-ungesättigte Carbonsäureamid, durchführt und die Umamidierung in Gegenwart von katalytischer Mengen Säure vornimmt.

2. Verfahren nach Anspruch 1, dadurch gekennzeichnet, daß das Molverhältnis 1 : 1 beträgt.

3. Verfahren nach Anspruch 1, dadurch gekennzeichnet, daß man als Polyhydroxiverbindungen aliphatische, gerad- oder verzweigtkettige, mehrwertige Alkohole, die gegebenenfalls Heteroatome enthalten können, verwendet.

4. Verfahren nach Ansprüchen 1 bis 3, dadurch gekennzeichnet, daß man als Polyhydroxyverbindungen aliphatische Diole mit 2 bis 6 Kohlenstoffatomen, bevorzugt 1,2-Ethandiol, aliphatische Triole mit 3 bis 6 C-Atomen, insbesondere Glycerin, Trimethylolpropan, Ethergruppen enthaltende mehrwertige Alkohole, bevorzugt Dihydroxyether, insbesondere Di-(2-Hydroxyethyl)-ether und/oder mehrwertige Aminoalkohole, bevorzugt Triethanolamin, einsetzt.

5. Verfahren nach den Ansprüchen 1 bis 4, dadurch gekennzeichnet, daß man die Michael-Addition im Temperaturbereich von 20 bis 70° C, bevorzugt 40 bis 50° C durchführt.

6. Verfahren nach den Ansprüchen 1 bis 5, dadurch gekennzeichnet, daß man in der Michael-Addition α,β-ungesättigte Carbonsäureamide der allgemeinen Formel

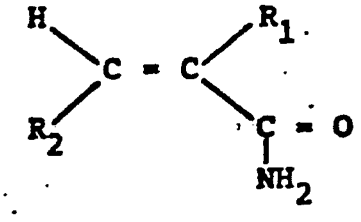

einsetzt, in der $R_1$ und $R_2$ Wasserstoff oder Methyl bedeuten.

7. Verfahren nach den Ansprüchen 1 bis 6, dadurch gekennzeichnet, daß man die Umamidierung mit Aminen der Formel

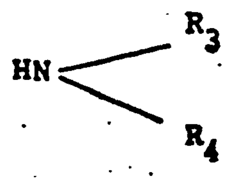

durchführt, worin
$R_3$ Wasserstoff und
$R_4$ den Rest -Q-$R_5$ bedeuten, wobei
Q für einen gegebenenfalls substituierten, gerad- oder verzweigtkettigen oder aus 5- bis 6-gliedrigen iso- oder heterocyclischen Ringen bestehenden oder solche Ringe enthaltenden organischen Rest mit 2 bis 18 C-Atomen steht
oder
$R_3$ und $R_4$ jeweils einen Alkylrest mit 1 bis 5 Kohlenstoffatomen bedeuten, wobei $R_3$ und $R_4$ auch zusammen einen 5- oder 6-gliedrigen iso- oder heterocyclischen Ring bilden können und wobei
$R_5$ für Wasserstoff, eine Dialkylamino- oder Alkoxygruppe steht, deren Alkylrest jeweils 1 bis 4 Kohlenstoffatome enthalten.

8. Verfahren nach den Ansprüchen 1 bis 7, dadurch gekennzeichnet, daß man die Umamidierung in Gegenwart katalytischer Mengen einer Carbonsäure, vorzugsweise Acrylsäure, vornimmt.

9. Verfahren nach den Ansprüchen 1 bis 8, dadurch gekennzeichnet, daß man die Umamidierung bei Temperaturen vornimmt, die im Bereich von 100 bis 180°C liegen.

10. Verfahren nach den Ansprüchen 1 bis 9, dadurch gekennzeichnet, daß man die Pyrolyse des umamidierten Michael-Addukts bei Temperaturen von wenigstens 150°C, vorzugsweise 150 bis 300°C durchführt.

11. Verfahren nach den Ansprüchen 1 bis 10, dadurch gekennzeichnet, daß man die Umamidierung und Pyrolyse unter Inertgas, vorzugsweise Stickstoff vornimmt.

12. Verfahren nach den Ansprüchen 1 bis 11, dadurch gekennzeichnet, daß man die Pyrolyse im Vakuum, vorzugsweise bei Drucken von 10 bis 100 hPa und die Fraktionierung im Vakuum, gegebenenfalls gleichzeitig durchführt.

**Claims**

1. A process for the production of N-substituted, α,β-unsaturated carboxylic acid amides by the reaction of an α,β-unsaturated carboxylic acid amide that is unsubstituted at the amido nitrogen, by basic catalysis in accordance with the Michael addition with an alcohol, transamidation of the resulting Michael adduct with a primary or secondary amine with elimination of ammonia to form an N-substituted carboxylic acid amide with a protected double bond, and subsequent pyrolytic removal of the alcohol, characterized in that the Michael addition is carried out with an organic polyhydroxy compound containing at least two alcoholic OH-groups, and having a boiling point of $\geqq$ 150°C at 1010 hPa in a molar ratio of 0.75 : 1 to 2 : 1 relative to the α,β-unsaturated carboxylic acid amide, and that the transamidation is carried out in the presence of catalytic amounts of acid.

2. A process according to claim 1, characterized in that the molar ratio is 1 : 1.

3. A process according to claim 1, characterized in that aliphatic straight or branched chain polyvalent alcohols that optionally may contain heteroatoms are used as the polyhydroxy compounds.

4. A process according to claims 1 to 3, characterized in that the polyhydroxy compounds are aliphatic diols having 2 to 6 carbon atoms, preferably 1,2-ethane diol, aliphatic triols with 3 to 6 carbon atoms, particularly glycerine, trimethylol propane, polyvalent alcohols containing ether groups, preferably dihydroxy ethers, particularly di-(2-hydroxy ethyl)-ether and/or polyvalent aminoalcohols, preferably triethanol amine.

5. A process according to claims 1 to 4, characterized in that the Michael addition is carried out in a temperature range from 20 to 70°C, preferably 40 to 50°C.

6. A process according to claims 1 to 5, characterized in that α,β-unsaturated carboxylic acid amides of the general formula

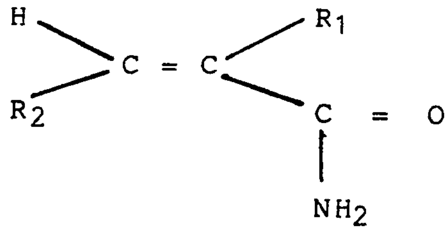

are used in the Michael addition, wherein $R_1$ and $R_2$ represent hydrogen or methyl.

7. A process according to claims 1 to 6, characterized in that the transamidation is carried out with amines of the general formula

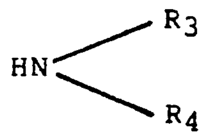

wherein

$R_3$ represents hydrogen and

$R_4$ the -Q-$R_5$ radical, wherein

Q represents an optionally substituted straight or branched chain organic radical having 2 to 18 carbon atoms, or which organic radical consists of 5- to 6-membered isocyclic or heterocyclic rings, or which contains such rings,

or

$R_3$ and $R_4$ each represent an alkyl group having 1 to 5 carbon atoms, wherein $R_3$ and $R_4$ can together form a 5- or 6-membered isocyclic or heterocyclic ring, and in which

$R_5$ represents hydrogen, a dialkylamino group or alkoxy group, the alkyl group of each containing 1 to 4 carbon atoms.

8. A process according to claims 1 to 7, characterized in that the transamidation is carried out in the presence of catalytic amounts of a carboxylic acid, preferably acrylic acid.

9. A process according to claims 1 to 8, characterized in that the transamidation is carried out at temperatures in the range of 100 to 180° C.

10. A process according to claims 1 to 9, characterized in that the pyrolysis of the transamidated Michael adduct is carried out at temperatures of at least 150° C, preferably 150 to 300° C.

11. A process according to claims 1 to 10, characterized in that the transamidation and pyrolysis are carried out under inert gas, preferably nitrogen.

12. A process according to claims 1 to 11, characterized in that the pyrolysis is carried out under vacuum, preferably at pressures of 10 to 100 hPa and the fractionation is carried out under vacuum, pyrolysis and fractionation optionally being carried out simultaneously.

## Revendications

1. Procédé de préparation d'amides d'acides carboxyliques à insaturation α,β N-substitués par une réaction d'addition de Michael, catalysée par une base, d'un amide carboxylique à insaturation α,β non substitué sur l'azote de la fonction amido avec un alcool, transamidation du produit d'addition selon Michael formé à l'aide d'une amine primaire ou secondaire, avec élimination concomitante d'ammoniac, en amide carboxylique N-substitué à double liaison protégée et élimination subséquente de l'alcool par pyrolyse, caractérisé en ce que la réaction d'addition de Michael est effectuée avec un composé organique polyhydroxylé, contenant au moins deux groupes OH alcooliques et possédant un point d'ébullition égal ou supérieur à 150°C sous 1010 hPa, dans un rapport molaire compris entre 0,75 : 1 et 2 : 1 par rapport à l'amide carboxylique α,β-insaturé et la transamidation est entreprise en présence de quantités catalytiques d'un acide.

2. Procédé suivant la revendication 1, caractérisé en ce que le rapport molaire est de 1 : 1.

3. Procédé suivant la revendication 1, caractérisé en ce que les composés polyhydroxylés sont choisi parmi les alcools aliphatiques à chaîne droite ou ramifiée polyvalents, pouvant éventuellement contenir des hétéroatomes.

4. Procédé suivant les revendications 1 à 3, caractérisé en ce que les composés polyhydroxylés sont choisis parmi les diols aliphatiques en $C_2$ à $C_6$, en particulier l'éthane-diol-1,2, les triols aliphatiques en $C_3$ à $C_6$ en particulier la glycérine et le triméthylolpropane, les alcools polyvalents à groupe éther, de préférence les éthers dihydroxyliques et en particulier l'éther di-(hydroxy-2 éthylique) et(ou) les aminoalcools polyvalents, en

particulier la triéthanol-amine.

5. Procédé suivant les revendications 1 à 4, caractérisé en ce que la réaction d'addition de Michael est réalisée dans la gamme de températures de 20 à 70° C et de préférence entre 40 et 50° C.

6. Procédé suivant les revendications 1 à 5, caractérisé en ce que la réaction d'addition de Michael est réalisée avec des amides carboxyliques α,β-insaturés de la formule

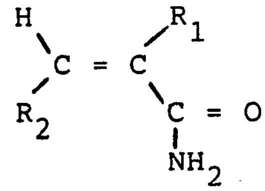

dans laquelle $R_1$ et $R_2$ désignent des atomes d'hydrogène ou des radicaux méthyle.

7. Procédé suivant les revendications 1 à 6, caractérisé en ce que la transamidation est réalisée avec des amines de la formule

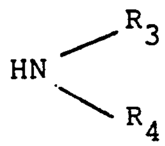

dans laquelle

$R_3$ désigne un atome d'hydrogène et

$R_4$ un groupe -Q-$R_5$, dans laquel

Q représente un groupe organique en $C_2$ à $C_{18}$, à chaîne droite ou ramifiée, éventuellement substitué, ou formé de ou contenant des noyaux iso- ou hétéro-cycliques penta- ou hexagonaux,

ou

$R_3$ et $R_4$ désignent chacun un radical alkyle en $C_1$ à $C_5$ ou forment ensemble un noyau iso- ou hétro-cyclique penta- ou hexagonal, et

$R_5$ désigne un atome d'hydrogène ou un groupe alcoxy ou dialkyl-amino avec des radicaux alkyle en $C_1$ à $C_4$.

8. Procédé suivant les revendications 1 à 7, caractérisé en ce que la transamidation est réalisée en présence d'une proportion catalytique d'un acide carboxylique, de préférence d'acide acrylique.

9. Procédé suivant les revendications 1 à 8, caractérisé en ce que la transamidation est effectuée à des températures comprises dans la gamme de 100 à 180° C.

10. Procédé suivant les revendications 1 à 9, caractérisé en ce que la pyrolyse du produit d'addition selon Michael est réalisée à des températures supérieures à 150° C, en particulier entre 150 et 300° C.

11. Procédé suivant les revendications 1 à 10, caractérisé en ce que la transamidation et la pyrolyse sont réalisées sous une atmosphère d'un gaz inerte, de préférence d'azote.

12. Procédé suivant les revendications 1 à 11, caractérisé en ce que la pyrolyse est réalisée sous vide, de préférence sous des pressions de 10 à 100 hPa, et le fractionnement est également effectué sous vide, le cas échéant simultanément.